# EUROPÄISCHE PATENTSCHRIFT

(11) **EP 0 327 612 B1**
(45) Veröffentlichungstag und Bekanntmachung des Hinweises auf die Patenterteilung: **03.05.1995**
(21) Anmeldenummer: 88905695.8
(22) Anmeldetag: 08.07.1988
(51) Int. Cl.: A61K 38/06

(54) **PHARMAZEUTISCH THERAPEUTISCHE VERWENDUNG VON GLUTATHION-DERIVATEN**
PHARMACEUTICAL THERAPEUTIC USE OF GLUTATHIONE DERIVATIVES
UTILISATION PHARMACEUTIQUE THERAPEUTIQUE DES DERIVES DE GLUTATHION

(30) Priorität: 09.07.1987 DE 3722647
(43) Veröffentlichungstag der Anmeldung: 16.08.1989
(60) Teilanmeldung: 94108938.5
(73) Patentinhaber: Ohlenschläger, Gerhard, Dr. med., 61462 Königstein (DE)
(72) Erfinder: Ohlenschläger, Gerhard, Dr. med., 61462 Königstein (DE)
(74) Vertreter: Lieck, Hans-Peter, Dipl.-Ing.
(86) Internationale Anmeldenummer: DE8800427
(87) Internationale Veröffentlichungsnummer: WO8900427

(56) Entgegenhaltungen:
- GB-A- 144 404
- Chemical Abstract, vol. 81, 1974, p. 161-162, Abstract number 116020h
- Biochemical andBiophysical Research Communications, Band 35, Nr. 5, 1969, R.Vince et al., Seiten 593-598, Seiten 593, 594, 1. Absatz, Seite 596, Tabelle 1; Seite 597, Zeilen 13-16
- Science, Band 212 (4494), 1. Mai 1981, A.M. Novi, Seiten 541-542, siehe ganzen Artikel; Seite 542, letzter Absatz
- Cancer Research, Band 47, Nr. 2, 15. Jan. 1987, J.P. Perchellet et al., Seiten 477-485, siehe Zusammenfassung; Seite 481, Tabellen 4,5; Seite 482, Tabelle 7
- Archives of Biochemistry and Biophysics, Band 239, Nr. 2, Juni 1985, Seite 546-548
- J. Lab. Clin. Med., Band 105, 1985 E. Beutler et al, Seiten 581-584, siehe Zusammenfassung; Seite 582, Figur 2; Seite 583, Tabelle 1
- Proc. Natl. Acad. Sci. USA, BAnd 80, September 1983, Seiten 5258-5260
- Reynolds, JEF, Prasad AB "Martindale" - The Extra Pharmacopoeia, 1982, Pharm.Press, London, siehe Seite 53

## Beschreibung

Alle höherorganisierten vielzelligen lebenden Systeme, auch der Mensch,decken den Energiebedarf ihrer Zellen und Gewebe nach dem Prinzip der biologischen Oxidation.

Kohlenhydrate, Aminosäuren, Fettsäuren und Nukleotide geben bei Dehydierung (Dehydrierung = Oxydation) in verschiedenen Stoffwechselwegen einen Teil der in diesen Molekülen gespeicherten chemischen Energie ab und werden schließlich dem gemeinsamen Endabbau im Zitronensäurezyklus zugeführt.

Im Stoffwechselzyklus gesunder Zellen bzw. Gewebe, der die gemeinsame Endstufe für Monosaccharide, Fettsäuren und Aminosäuren darstellt, werden durch zwei Dekarboxylierungsreaktionen die in den Zyklus eintretenden C-6-Verbindungen (Zitronensäure) während eines Umlaufs im Zyklus zu C-4-Verbindungen (Oxalessigsäure) abgebaut, die Kohlenstoffkette wird also um zwei Kohlenstoffatome verkürzt.

Außerdem werden im Zitronensäurezyklus 8 H-Atome über die Atmungskette unter Energiegewinn zu vier Molekülen Wasser oxidiert.

Der auf den verschiedenen Abbauwegen bei Dehydrierungen anfallende Wasserstoff wird von Wasserstoff-übertragenden Coenzymen NAD und FAD zu der Innenmembran der Mitochondrien transportiert, den Zellorganellen, die als Kraftwerke einer Zelle gelten. Der Wasserstoff bildet mit strukturgebundenen Enzymen der Atmungskette, den Cytochromen, eine Elektronenrtransportkette, die - wie auch der gesamte Stoffwechsel - nur funktionieren kann, wenn im letzten Glied der Kette der Wasserstoff in einer Redoxreaktion mit Sauerstoff zu Wasser reagieren kann. Das Normalpotential Eₒ dieser Redoxreaktion liegt bei einem pH-Wert von 7 bei + 0,810 Volt, so daß auf die Elektronentransportkette ein Sog ausgeübt wird.

Sauerstoff ist im Stoffwechselprozess also der terminale Wasserstoff- bzw. Elektronenakzeptor und hält durch seine Anwesenheit den Elektronenfluß im Stoffwechsel einer Zelle aufrecht.

Die Atmungskette in den Mitochondrien führt somit eine Art biochemische Knallgasreaktion aus, wobei die Atmungssubstrate dehydrogeniert werden. Durch die stufenweise Reaktion des Wasserstoffs bzw. der Elektronen über eine Kaskade von Zwischenträgern wird die bei der Wasserbildung aus Wasserstoff und Sauerstoff abgegebene hohe Reaktionsenthalpie allmählich freigesetzt und ein beträchtlicher Anteil in Form chemischer Energie als Adenosintriphosphat (ATP) für energieverbrauchende Zellreaktionen gespeichert.

Jede Mitochondrien aufweisende gesunde Zelle deckt auf diese Weise ihren Energiebedarf. In Abhängigkeit von den Stoffwechselaufgaben variiert die Anzahl der Mitochondrien zwischen 10² und 6 x 10³ einer Zelle, entsprechend unterschiedlich ist auch deren Sauerstoffbedarf.

Sauerstoff muß zu jeder Sekunde im Leben eines oxidierend lebenden Systems in ausreichendem Maße in den Mitochondrien aller Zellen zur Verfügung stehen. Dazu erforderlich ist ein ständiger ungestörter Sauerstoffstrom von der Atemluft mit ausreichendem Sauerstoffpartialdruck über die Atmungswege, den Übertritt des Sauerstoffs an der Lungen-Blutschranke der Lungen-Alveolen, die Diffusion durch die Erythrozytenmembran, die Hämoglobinbildung und Loslösung vom Hämoglobin in den Blutkapillaren des Gewebes, die Sauerstoffdiffusion durch den Zwischenzellenraum, den interstitiellen Raum, durch die Zellmembran und die Mitochondrienmembran bis hin zur oben beschriebenen Atmungskette. Ein weiter, aber wichtiger Weg des Sauerstoffs.

Diese ständig ungestört ablaufende Oxidation in allen Zellen ist die wichtige Voraussetzung für die Gesunderhaltung des Organismus. Im annäherungsweisen Vergleich mit elektrischen oder elektronischen Bauelementen und deren Schaltkreisen kann von einer Arbeitskennlinie einer gesunden Zelle gesprochen werden. Auf dieser ist ein optimaler Arbeitspunkt vorhanden, der sich nur geringfügig auf der Arbeitskennlienie verschieben darf. Ist der Arbeitspunkt unzulässig weit von seinem Optimum entfernt, so ist dies ein Anzeichen für eine beträchtliche Störung des Stoffwechsels der Zelle. Eine derartige Störung wird beispielsweise durch das Phänomen der Überoxidation verursacht, welches auch als oxidativer Stress bezeichnet wird. Der oxidative Stress kann entweder durch ein Überangebot von aktivierten Sauerstoff-Stufen (Sauerstoff-Radikale) und/oder durch eine Verminderung jener Moleküle entstehen, welche normalerweise diese Radikalenergie abzufangen vermögen und als Scavengermoleküle bezeichnet werden. Sind die Zellen nicht aus sich heraus imstande, die Störung zu beseitigen, so kommt es zu einer Zerstörung von Biomolekülen und Zellstrukturen wie Lipidperoxidation der Zellmembranen, zu vielfältigen Zellfunktionsstörungen, zur krebsartigen Entartung einer Zelle und schließlich zum Zelluntergang.

Die Natur mußte in lebenden Systemen von Anfang an ihre molekularen und Zellstrukturen vor der Einwirkung der zerstörerischen Sauerstoff-Radikale, die durch exogene Noxen und auch im Zellstoffwechsel ständig entstehen, schützen und entwickelte Schutzenzyme und Schutzmoleküle als Scavenger, die diese Radikale sicher und schnell abfangen können.

Ist es den einzelnen gestörten Zellen nicht möglich die energiereichen Radikale abzufangen, so können in der Folge Krankheiten entstehen, wie Krebs jeder Genese, maligne Erkrankungen der Blutzellen, Hepatopathien, Fettleber, Fettzirrhose, Leberzirrhosen jeder Genese, Störungen der immunologischen Abwehrfunktionen im Bereich der Natural-Killer-Zellen, komplexe Störungen der Lymphokin-Biosynthese in T-Helferzellen, Cardiomyopathien jeder Genese, neurologische Erkrankungen entzündlicher, allergischer oder degenerativer Genese, Blutzellerkrankungen, Augenlinsenschäden, Proliferationsstörungen und Differenzierungsstörungen von Epithel-Endothel- und Schleimhautgeweben und viele mehr. Welche Art der Erkrankung eintreten wird, hängt im wesentlichen davon ab, welche Zellen oder Zellgewebe am schwersten betroffen sind.

Hieraus ergibt sich, daß die in lebenden Systemen zwangsläufig notwendigen Oxidationen nicht zu Überoxidationen führen dürfen.

### Chemisch gesehen bedeutet Oxidation:

1. Abgabe von Elektronen.
2. Abgabe von Wasserstoff.
3. Aufnahme von Sauerstoff.

### Reduktion bedeutet entsprechend:

1. Aufnahme von Elektronen.
2. Aufnahme von Wasserstoff.
3. Abtgabe von Sauerstoff.

Thermodynamisch betrachtet sind alle Reduktionen endotherme Prozesse, alle Oxidationen dagegen exotherme. Das bedeutet, daß die oxidierte Stufe "energieärmer", die reduzierte Stufe "energiereicher" ist. Stets kann die Red-Stufe "Energie" = Elektronen abgeben und die Ox-Stufe wird durch "Energieaufnahme", durch Aufnahme von Elektronen reduziert.

Die Basis für alle reduktive Potenz im Blut und in den meisten Zellen von Mensch und Säugetieren ist das Tripeptid Glutathion in seiner reduzierten Form (G-SH) bestehend aus den drei Aminosäuren Glutaminsäure, Cystein und Glycin mit folgender Strukturformel: 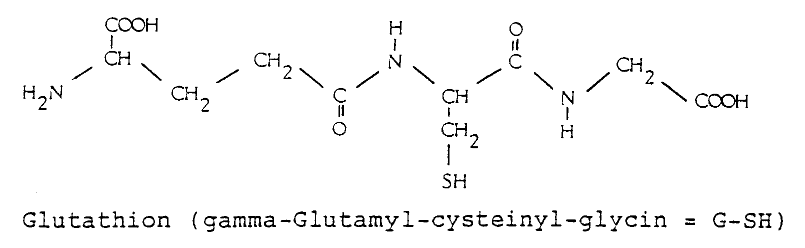
Die Funktionalität lebender Systeme, die auf dem Prinzip der biologischen Oxidation beruht, ist nur möglich durch das Bestehen eines reduktiven Schutzes durch dieses wichtige Molekül: G-SH.

Durch Dehydrierung bzw. durch Oxidation können zwei Moleküle reduziertes Glutathion (G-SH) unter Disulfidbildung in ein Molekül oxidiertes Glutathion (G-S-S-G) übergehen.

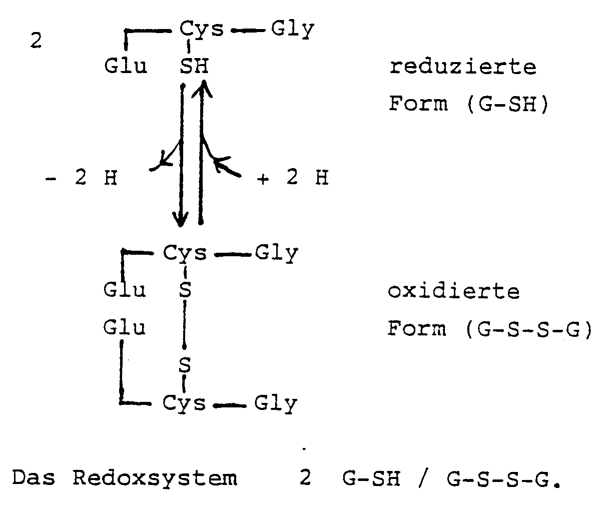

In zahlreichen Untersuchungen konnte festgestellt werden, daß Zellen, die ihrer spezifischen Funktion einwandfrei nachkommen, ein Konzentrationsverhältnis von reduziertem Glutathion (G-SH) zu oxidiertem Glutathion (G-S-S-G) von etwa 400 zu 1 aufweisen. Das heißt, die intakte lebensfähige Zelle besitzt ein hohes reduktives Potential in Form des Gehaltes an reduziertem Glutathion. Das Konzentrationsverhältnis von G-SH zu G-S-S-G im Bereich von 400 zu 1 kann als Indiz für optimale Funktion und sichere Strukturerhaltung gewertet werden.

Zur Erhaltung der Funktionalität vieler, vielleicht aller Enzyme des Zellstoffwechsels, zur Verhinderung oxidativer Veränderungen ihrer katalytischen und allosterischen Zentren, zur Erhaltung einer optimalen Konformation ist das reduktive Potential des reduzierten Glutathions, d.h. seine optimale, hohe intrazelluläre Konzentration von äußerster Wichtigkeit.

Alle zur Zeit bestehenden klassischen Therapien stützen entweder das Phänomen der Oxidation, also die oxidative Seite lebender Systeme, oder greifen durch Arzneimittel, die als Xenobiotika zu ihrem Abbau und zur Metabolitbildung der Oxidation bedürfen, in die "Oxidative Potenz" des Stoffwechsels ein.

Allerdings sind auch schon das reduzierte Glutathion selber sowie bestimmte Derivate des Glutathions auf ihre Eignung als therapeutisch wirksame Substanzen untersucht worden. Die GB-A-144 024 schlägt einen krebshemmenden Nahrungs- oder Futtermittelzusatz vor, der aus einer Reihe als wesentlich bezeichneter Stubstanzen besteht, von denen eine Glutathion sein kann. Die Zeitschrift Cancer Res., 15.01.1987, Vol. 47(2), Seiten 477-485 enthält eine Untersuchung, wie die Behandlung mit reduziertem Glutathion die Entwicklung von Tumoren beeinflußt. In der Zeitschrift Biochemical and Biophysical Research Communications, Band 35, Nr. 5, 1969, R. Vince et al.: "Glyoxalase inhibitors as potential anticancer agents", Seiten 593-598, ist eine invitro-Versuchsreihe mit S-Alkylglutathion-Derivaten zur Hemmung des Enzyms Glyoxalase I beschrieben. Es handelt sich um ein Enzym, welches Methylglyoxal zu S-Lactoylglutathion verbindet. Bei den in-vitro-Versuchen kommt es zu unterschiedlich starken Hemmungen des Enzyms. Es wird jedoch nicht mitgeteilt, welche pharmazeutische Relevanz diese Hemmung haben könnte.

In der Zeitschrift Archives of Biochemistry and Biophysics, Band 239, Nr. 2, Juni 1985, Academic Press, Inc. (US), ist dargestellt, daß Glutathion für den Zellen-Metabolismus wesentlich ist und die Zelle gegen freie Radikale, oxydative Kräfte und Intoxikation schützt. Es wird auch schon erkannt, daß Glutathion selbst eine schlechte Bioverfügbarkeit hat, also nicht leicht in die Zellen gelangt. Dem werden zwei Derivate des Glutathions, nämlich Glutathion-Monomethyl-Ester und Glutation-Monoethyl-Ester gegenübergestellt, die eine bessere Bioverfügbarkeit haben und in der Zelle zu u.a. Glutathion zerfallen, so daß eine Gabe dieser Derivate zu erhöhtem Glutathion-Spiegel in den Zellen führt. Es sind dies Derivate, bei denen Glycin-Karboxyl-Gruppe des Glutathions verestert ist.

Der Erfindung liegt die Aufgabe zugrunde, Glutathion-Derivate zur Verfügung zu stellen, die, verwendet als Arzneimittel, geeignet sind, das Redoxpotential der im Stoffwechsel gestörten Zellen zu stärken oder gar zu monopolisieren, und dabei eine gute Bioverfügbarkeit haben.

Zur Lösung dieser Aufgabe werden erfindungsgemäß zwei Glutathion-Thiol-Derivate zur Verfügung gestellt, die in Anspruch 1 gekennzeichnet sind, nämlich der Mono-Acetyl-Glutathionyl-(Thio)-Ester und der Mono-Phosphorsäure-Glutathionyl-(Thio)-Ester des Glutathions. Mit besonderem Vorteil werden diese Derivate in Kombination mit weiteren Substanzen gemäß den Ansprüchen 2 bis 4 als Arzneimittel verwendet.

Das erstgenannte S-Acetyl-Glutathion ist - ohne Angabe einer pharmazeutischen Wirksamkeit - aus Chemical Abstracts, Band 81, 1974, Zusammenfassung Nr. 116020 H vorbekannt.

Durch die zentrale molekularbiologische Bedeutung des reduzierten Glutathions in physiologisch angemessener, intrazellulärer Konzentration sind die folgenden physiologischen, biochemischen und vitalen Zellfunktionen alle als vom intrazellulären Glutathionstatus (G-SH > gemischte Disulfide > G-S-S-G) allein abhängig erklärbar.
· Redoxpotential.
· Optimale Arbeitsfähigkeit aller Enzymreaktionen (Aktives Zentrum sowie allosterische Zentren eines Enzymmoleküls).
· Bewahrung der Membranintegrität aller biologischer Membranen (Zell- und Zellorganell-Membranen).
· Dies ist auch besonders wichtig zur Funktionsoptimierung strukturgebundener Enzyme,für Membran-Carriermechanismen,für die Funktion und allosterische Spezifität aller Zellrezeptoren.
· Cofaktor und Reduktionspotential vieler Enzyme,vor allem solcher,die in die Entgiftung eingeschaltet sind.
· Zur Regulation und Regulationsnormalisierung des komplexen Raum-Zeit-Musters von Zellwachstums- und Zelldifferenzierungs-Prozessen.
· Zur Verhinderung,Abschwächung und Terminierung radikalischer Reaktionen und radikalischer Kettenreaktionen in lebenden Systemen.

So ist die physiologisch intrazelluläre Konzentration an reduziertem Glutathion primäre Voraussetzung für alle grundlegenden Zellfunktionen.

Und deshalb ist die Normalisierung der intrazellulären G-SH-konzentration zur Therapie und zur Prophylaxe vieler Erkrankungen, der meisten Erkrankungen,auch unterschiedlicher Ätiologie und durch phänomenologisch unterschiedliche pathobiochemische Noxen von kausal-therapeutischer Bedeutung.

Eine sichere Basistherapie zur Normalisierung eines durch unterschiedlichste Noxen geschädigten Zellstoffwechsels wird durch die gut bioverfügbaren und wirksamen mit der Erfindung vorgeschlagenen Glutathion-Derivate:
Mono-acetyl-(thio)-ester des gamma-Glutamyl-cysteinylglycin,
Mono-phosphorsäure -(thio)-ester des gamma-Glutamyl-cysteinyl-glycin
erstmalig möglich.

Wesentliche Vorteile bei der pharmazeutisch,therapeutischen Anwendung der hier neu beschriebenen Derivate des reduzierten Glutathions (G-SH) bei Erkrankungen von Mensch und Tier sind folgende:
A) Die gute Permeationsfähigkeit dieser Derivate durch biologische Membranen,wodurch erst eine wirkungsvolle intrazelluläre Therapie mit Glutathion mit allen, ihren positiven physiologischen Konsequenzen möglich wird.
B) Schutz der für die therapeutischen Effekte so wichtigen SH-Gruppe des Glutathions auf dem Weg durch biologische Kompartimente bis hin zum gewünschten Wirkort.
C) Keine Hemmung der in die endogene Glutathion-Biosynthese eingeschalteten Enzyme (gamma-Glutamylcystein-Synthetase,das Regulationsenzym durch Glutathion im Sinne eines negativen feed-back; Glutathion-Synthetase).
   Dadurch besteht die Möglichkeit einer Glutathion- (G-SH) Substitution für den intrazellulären Raum,ohne daß auf dem Wege einer kompetitiven und/oder allosterischen Hemmung eine je nach Grad der zellschädigung noch mögliche endogene Glutathion-Biosynthese gehemmt oder blockiert würde.
D) Die pharmazeutisch-therapeutische Anwendung der Glutathion-Derivate führt auch zu keiner Hemmung der endogenen Glutathion-Biosynthese in gesunden Zellen.

Damit eine therapeutisch nutzbare Glutathion-Therapie in therapeutisch wirksamen und vertretbaren intrazellulären Konzentrationen über unterschiedliche Applikationswege (oral-enteral,nasal, buccal,sublingual,per inhalationem,vaginal,rektal,intracutan, subcutan,intramuskulär,intravenös,per infusionem,intraarteriell) möglich ist,muß das Molekül mit intakter SH-Gruppe intrazellulär zur Wirkung kommen können und gut membrangängig sein.

Diese Wesentliche Anforderung für die rel. breite therapeutische Einsatzfähigkeit von Glutathion (G-SH) wird erstmalig durch die erfindungsgemäß vorgeschlagenen Derivate möglich.

Wegen der grundsätzlichen Wirkung von G-SH in geeigneten intrazellulären Konzentrationen zur Normalisierung basaler Mechanismen des Zellstoffwechsels können die beschriebenen Derivate alleine und/oder in Kombination mit sich selbst oder mit Präparaten gemäß den Ansprüchen 2 - 4 eingesetzt werden:
Bei allen entzündlichen (auch allergischen und autoaggressiven), chemisch-toxischen, physikalisch-toxischen (auch Strahlenschäden durch elektromagnetische Wellen und/oder Korpuskularstrahlung) infektiös-toxischen Zell-, Gewebe- und Organschäden;
sowie bei pathologischen Einlagerungen,Ablagerungen an und ins Gewebe;bei Atrophien,Hypertrophien,Dys- bis Anaplasien,also bei gutartigen Tumoren und bei malignen Neoplasien mit und ohne Metastasierung.
Bei Denaturierungsprozessen an und mit biologischen Molekülen, in normotrophen und bradytrophen Geweben.
Bei der Immunschwäche jeder Genese.
Zur Krebsprophylaxe und zur adjuvanten Krebsbehandlung im Rahmen anderer Krebstherapien und solchen mit Chemotherapeutika oder mit einer Strahlenbehandlung.

Außerdem kann die durch Mangel an reduziertem Glutathion bedingte Zunahme der Sauerstoff-Radikale optimal abgefangen werden, welche die Molekül- und Zellstruktur zerstören und somit maligne Entartungen oder den Zelltod herbeiführen können.

Die Verhinderung einer "Überoxidation" durch ein physiologisches Einstellen des Redoxpotentials von Zellen und Geweben unter Verwendung der vorgeschlagenen Thiol-Derivate des reduzierten Glutathions als Therapeutikum ist ein grundsätzlich neuer Ansatz zur Behandlung vieler Erkrankungen einschließlich der Prophylaxe und Behandlung maligner Tumore.

Die Glutathion-Reduktase, ein Flavoprotein, das die Funktion des Elektronenüberträgers erfüllt, stellt in allen menschlichen Zellen ein Konzentrationsverhältnis von (G-SH) zu (G-S-S-G) von etwa 400 : 1 ein. Dieser Wert spiegelt die Vitalität bzw. die physiologische Funktionsfähigkeit einer Zelle wider.

Die Biosynthese von Glutathion aus den drei Aminosäuren Glutaminsäure, Cystein und Glycin erfolgt intrazellulär DNA-unabhängig unter Einschaltung von zwei Enzymen, der gamma-Glutamyl-cystein-Synthetase und der Glutathion-Syntethase unter Verbrauch von 2 Mol Adenosin-triphosphat (ATP). 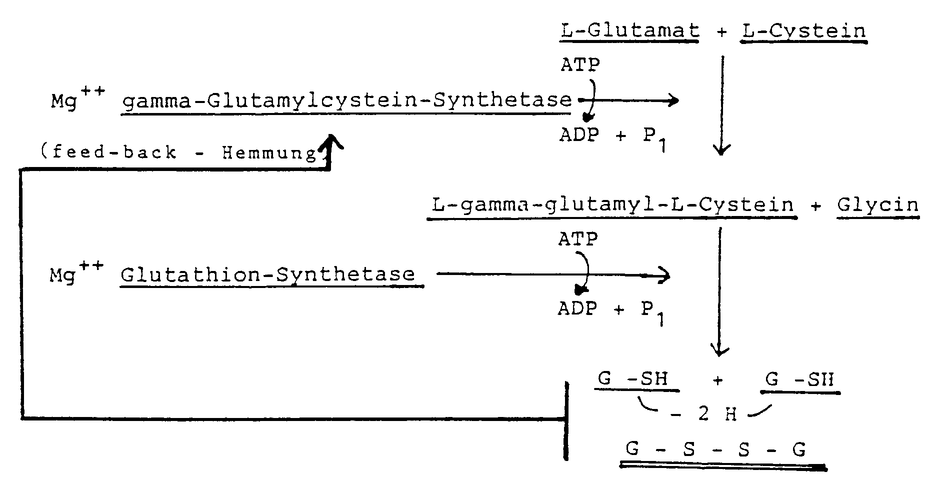
Schematischer Ablauf der intrazellulären Glutathion-Biosynthese.

Die relativ ungewöhnliche Peptidbindung über die gamma-Carboxylgruppe der Glutaminsäure verhindert einen unkontrollierten Abbau des Glutathionmoleküls durch Peptidasen.

Die SH-Gruppen des reduzierten Glutathions (G-SH) sind sehr reaktive Radikal-Scavenger, indem sie ihren Wasserstoff an Kohlenstoff-, Sauerstoff- und Stickstoff-Radikale abgeben. Neben der Funktion des G-SH / G-S-S-G Systems als grundlegendes Redoxsystem lebender Systeme hat der zuvor angesprochene Wasserstoff-Übertragungsaspekt der reaktiven SH-Gruppe eine besonders wichtige Bedeutung für die umfassende biologische Rolle des G-SH.

Mögliche Ein-Elektron-Übertragungsprozesse sind nachfolgend dargestellt:
A. Wasserstoffentzug Thiyl-Radikal

   RSH + R·' → RS· + R'H

   (RS· + RS· → RSSR)
B. Photo-Ionisation

   RS⁻ → RS + e⁻
C. Ein-Elektron-Transfer zu einem Metallion

   RS⁻ + M²⁺→RS· + M⁺
D. Ein-Elektron-Reduktion von Disulfiden 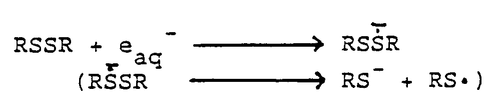
Auch Zwei-Elektronen-Übertragungen sind möglich, so z.B. wenn zwei G-SH in ein G-S-S-G übergehen.

In den verschiedenen menschlichen Geweben ist die biologische Halbwertszeit des Glutathions unterschiedlich. Im Erythrozyten ca. 100 Std., im Gehirn ca. 70 Std., in der Augenlinse ca. 30 Std. und in der Leber nur ca. 4 Std. !

Neben den Ein- und Zwei-Elektronen-Übergängen sind auch beim Glutathion die Bildung gemischter Disulfide möglich und die Thiol-Disulfid-Austausch-Reaktion.

Glutathion ist Bestandteil vieler wichtiger Enzyme wie Glutathion-Peroxidase oder Glutathion-Reduktase. Die Glutathion-Peroxidase schützt intrazellulär beispielsweise vor vielen auf unterschiedlichen Wegen entstandenen Hydroperoxiden durch ihre Fähigkeit diese in engem Zusammenwirken mit Katalasen zu Wasser zu reduzieren

Die Selen-abhängige Glutathion-Peroxidase spaltet H₂O₂ sowie organische Peroxide (ROOH). Eine zweite selenunabhängige Glutathion-Peroxidase spaltet ausschließlich organische Peroxide.

Die Glutathion-Reduktase sorgt intrazellulär für das zuvor bereits angesprochene physiologische Konzentrationsverhältnis von G-SH zu G-S-S-G. Die intrazellulären G-SH-Konzentrationen liegen bei 1 - 50 x 10⁻⁴M; die intrazellulären G-S-S-G-Konzentrationen nur bei 6 - 200 x 10⁻⁶M.

Ungefähr 30% des G-SH liegen in Form gemischter Disulfide (Prot-S-S-G) vor.

Die Aufgaben von Glutathion im Intermediärstoffwechsel des Menschen sind insbesondere:
1.) Redox-System.
2.) Thiol-Disulfid-Austauschreaktion mit Enzymen und Strukturproteinen.
3.) Entgiftung alkylierender Arzneimittel und Chemikalien.
4.) Aminosäure-Transport durch Zellmembranen.
5.) Chelatbildung und -bindung.
6.) Schutz vor "freien" Radikalen; Energieabsorption "freier" Radikale.
7.) Reaktionspartner definierter Enzymreaktionen:
   Glutathion-Peroxidase,
   Glutathion-Reduktase;
   Glutathion-S-Transferasen, wie
   Glutathion-S-Aryltransferasen,
   Glutathion-S-Alkyltransferasen,
   Glutathion-S-Epoxidtransferasen sowie
   Glutathion-Dihydroascorbat-Reduktase u.a.

Unter Berücksichtigung der vorstehend aufgeführten Funktionen sind folgende Wirkungen des reduzierten Glutathions charakteristisch:
1.) Verbesserung aller spezifischen Zellfunktionen durch Normalisierung des Redoxpotentials und Optimierung des Schutzes vor Zerstörung wichtiger Biomoleküle und Zellstrukturen durch Überoxidation, sowie Schutz vor Lipidperoxidation u.a.
2.) Optimierung der Funktion vieler Zellenzyme durch Einstellung eines, für die Zelle optimalen Redoxpotentials, zum Teil über die Bildung gemischter Disulfide.
3.) Normalisierung der Mitosepotenz einer Zelle über die Einstellung des Redoxpotentials; dadurch tumorprotektiv und tumortherapeutisch wirksam.
4.) Durchführung von Enzymreaktionen, da diese nur ablaufen können, wenn ausreichend Glutathion zur Verfügung steht. Glutathion ist Bestandteil vieler definierter Enzyme wie Glutathion-Peroxidase, Glutathion-S-Transferasen, Glutathion-Dihydroaskorbat-Reduktase.
5.) Normalisierung aller physiologischen Zellfunktionen, die durch die Einwirkung des Glutathions möglich sind.

Die positiven Wirkungen des reduzierten Glutathions in der Zelle werden in besonders ausgeprägter und sicherer Weise durch Gaben des Monoacetylderivats des reduzierten Glutathions und der Monophosphorsäureesters des reduzierten Glutathions hervorgerufen: 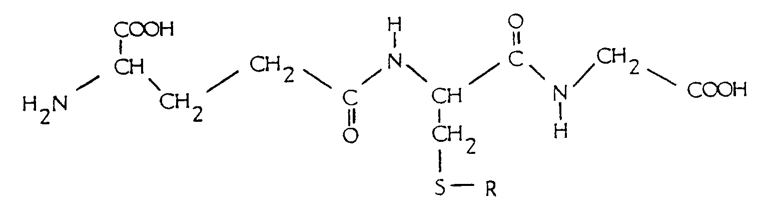
mit 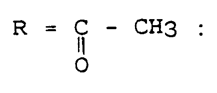
Mono-Acetyl-glutathionyl-(thio)-ester
oder R = PO₃H₂ : Mono-phosphorsäure-glutathionyl-(thio)-ester
Ausgezeichnete Verträglichkeit und gesteigerte Wirksamkeit zeigen Kombinationspräparate der Derivate in Verbindung mit Substanzen, welche Halbleiterelemente wie Silicium, Selen oder Germanium in organischer Bindung enthalten. Gleiches gilt für Kombinationspräparate mit Vitaminen wie Vitamin A, Vitamin E oder dem Provitamin Beta-Carotin sowie solche mit Aminosäuren wie L-Cystein oder L-Methionin. In derartigen Kombinationspräparaten können auch mehrere der genannten Substanzen enthalten sein. Derartige Präparate eignen sich zur Behandlung zahlreicher Krankheiten in einem weiten Indikationsrahmen.

### Indikationsgebiete

Behandlung von Krebserkrankungen jeder Genese, auch für maligne Erkrankungen der Blutzellen und ihrer Vorstufen.

Substitution und Stoffwechselregulation bei Durchführung anderer Krebstherapien, Chemotherapien, Strahlentherapien und/oder naturheilkundlicher Therapien.

Prophylaxe und Behandlung von Metastasen im Rahmen maligner Tumorerkrankungen.

Hepatopathien, insbesondere akute und chronische Erkrankungen an Hepatitis wie chemisch-toxische und infektiös-toxische Hepatitis, Virus-, Rickettsien-, Bakterien- oder Protozoenbedingte Hepatitiden sowie chronisch-aggressive Hepatitis, Fettleber, Fettzirrhose und Leberzirrhosen jeder Genese.

Störungen der immunologischen Abwehrfunktion im Bereich der Natural-Killer-Zellen, Monozyten, Makrophagen, Granulozyten, T- und B-Lymphozyten, Plasmazellen, sowie Störungen der Komplementfaktoren und Antikörpersynthese.

Komplexe Störungen der Lymphokin-Biosynthese in T-Helferzellen, Makrophagen und anderen Zellen.

Behandlung von Cardiomyopathien jeder Genese, auch in Verbindung mit anderen Therapien, alle Formen von Koronarleiden, Angina pectoris, Myokardinfarktprophylaxe sowie Notfalltherapie des Herzinfarktes in Verbindung mit anderen Notfallmedikamenten.

Erworbene und auch angeborene Formen von Skelettmuskelstörungen.

Neurologische Erkrankungen entzündlicher, allergischer oder degenerativer Genese.

Alle Formen von Blutzellerkrankungen, Anämien, Leukopenien, Lymphopenien und Thrombozytopenien.

Prophylaxe von Augenlinsenschäden, toxischen Störungen der Netzhaut und des Glaskörpers, sowie zur Kataraktprophylaxe.

Alle Formen der Überoxidation bzw. des oxidativen Stresses, beispielsweise im Rahmen der Anwendung von Sauerstoff-Therapien oder Therapien mit aktivierten Sauerstoff-Stufen (Sauerstoff-Radikale) sowie zum Schutz bei der Anwendung von hyperbarer Sauerstofftherapie, Sauerstoff- Mehrschritt-Therapie, bei Ozontherapien und HOT-Therapien.

Intoxikationen, die im menschlichen Organismus über radikalische Kettenreaktionen zu Biomolekül- und Gewebeschäden führen.

Begleitenden Therapie bei Bestrahlungsbehandlungen, Cytostatikabehandlungen sowie zur Abschwächung oder Verhinderung des Übelseins, der Nausea u.a.

Nach Narkosen, insbesondere nach Vollnarkosen bei herz- und lebergeschädigten Patienten.

Intoxikationen mit Xenobiotica, insbesondere mit toxischen Spurenelementen und mit Schwermetallen.

Proliferationsstörungen und Differenzierungsstörungen von Epithel-, Endothel- und Schleimhautgeweben.

Behandlung der pathophysiologischen und unterschiedlich entstehenden Arteriosklerose.

Basisbehandlung und adjuvante Behandlung von Allergien.

Behandlung der Impotentia coeundi und impotentia generandi, sowie Fertilitätsstörungen und Potenzstörungen jeder Genese. Auch bei vorzeitigem Altern,Altersverschleiß aller Gewebe u.zur Prophylaxe bei Betätigungen,die zu vorzeitigem Altern oder zu Organverschleiß führen.-

### Applikationsformen

Die vorgeschlagenen Thiol-Derivate des reduzierten Glutathions können zur Therapie in folgenden Formen appliziert werden:
1.) intravenös, auch als Infusion,
2.) intramuskulär,
3.) intracutan und subcutan,
4.) oral,
5.) nasal, buccal und sublingual,
6.) per inhalationem,
7.) vaginal und
8.) rektal.

Die intravenöse Injektion oder Infusion ist vorzugsweise bei schwerwiegenden und akut-bedrohlichen Erkrankungen empfehlenswert, wobei die Infusion insbesondere bei höherer Dosierung über einen längeren Zeitraum hinweg vorzuziehen ist.

Als Basis für die Bereitung der Infusionslösung eignen sich jeweils in reiner Form oder als Mischung physiologische Kochsalzlösung (0,9% NaCl in Wasser), Ringerlösung oder eine Monosaccharidlösung ( 5% Glucose, 5% Lävulose), wobei der pH-Wert der Lösung im Bereich zwischen 6,8 bis 7,4 liegt.

Die Aufbewahrung soll lichtgeschützt erfolgen, so daß für Ampullen dunkles Glas empfohlen wird.

Während bei der intravenösen Applikation dem Organismus eine Höchstmenge an Glutathion in reduzierter Form direkt in den Intravasal-Raum zugeführt werden kann, ist bei anderen Applikationsformen eine Teiloxidation der Substanz auf dem Resorptionsweg zu berücksichtigen.

Dennoch ist die subcutane, intramuskuläre, intraarterielle und auch die orale Darreichungsform bei Langzeitbehandlung von Vorteil. Die orale Darreichung ist in Form von Tabletten, Granulat, Kapseln, Pulver oder Tropfen möglich. Es versteht sich von selbst, daß auf dem oral-enteralen Aufnahmeweg eine Verminderung der Wirkung durch oxidative Reaktionen eintreten kann, wie dies auch beim Inhalat der Fall ist.

Sofern die medizinischen Voraussetzungen gegeben sind, ist die intratumorale und auch die intraperitoneale Applikation möglich.

Ebenfalls ist die Applikation in Form von Suppositorien möglich, also die rektale bzw. die vaginale Verabreichung mit der Einschränkung einer geringfügig schlechteren systematischen, aber relativ guten lokalen Bioverfügbarkeit.

In besonderen Fällen von schlecht heilenden Wunden wie Verbrennungen, Erfrierungen oder Gangrän-Ulzera kommt auch die Applikation in Form von Salben, Spray oder als Flüssigkeit in Frage und führt zu guten therapeutischen Ergebnissen.

Die unterschiedliche Dosierung und Galenik für die jeweilige Verabreichungsart entspricht der für jede Applikation unterschiedlichen Bioverfügbarkeit, biologischen Verträglichkeit, der therapeutischen Sicherheit und der dosisgerechten Unbedenklichkeit.

### Dosierung

Die therapeutische Dosierung, die sich im Tierversuch an Kleintieren wie Graumäusen, Wistar-Ratten und Kaninchen als untoxisch erwiesen hat, liegt für den Menschen in einem Bereich von 2 - 5 mg/kg Körpergewicht für eine Einzeldosis bei langsamer intravenöser Injektion, wobei das Injektionsvolumen 5 ml beträgt mit einem Gehalt an reduziertem Glutathion von 150 mg.

Als intravenöse Infusion können in einem Trägerolumen von 250 ml Tagesdosierungen von bis zu 20 mg/kg Körpergewicht und in besonders gelagerten Fällen auch noch höhere Dosierungen verabfolgt werden. Bei einer Dosierung von 20 mg/kg Körpergewicht sollte eine Mindest-Infusionszeit von 30 Minuten nicht unterschritten werden.

Injektions- und Infusionslösungen, welche reduziertes Glutathion enthalten, sollten mit anderen Arzneimitteln nicht gemischt werden. Infusionslösungen sind vor der Applikation stets frisch herzustellen, und sie sind zum alsbaldigen Verbrauch bestimmt.

Als Tagesdosierung wird grundsätzlich der Dosisbereich von 2 bis 20 mg/kg Körpergewicht empfohlen, wobei die genaue Dosis von den individuellen Gegebenheiten im Rahmen der Erkrankung abhängig ist. Einzig bei Krebserkrankungen mit Metastasen bei einer Gesamtmasse des Tumors von mehr als 100 g können höhere Tagesdosierungen als 20 mg/kg Körpergewicht Anwendung finden.

Die Dauer der Therapie richtet sich nach der zu behandelnden Grundkrankheit. Bei einer Therapiedauer von mehr als 4 Wochen sollte der Spurenelement-Serumspiegel kontrolliert werden. Zur quantitativen Erfassung wird empfohlen, den Serum-Zinkgehalt als Screeningtest-Parameter zu bestimmen.

Als therapeutische Leitparameter der Serumelektrolyten können die Gehalte an Kalium und Magnesium dienen.

### Galenik

Die injizierbaren Lösungen der Thiol-Derivate den reduzierten Glutathions sollten als schwermetallfreie Lösungen in sterilem und pyrogenfreiem Wasser mit einem Leitfähigkeitswert von weniger als 5 »S vorliegen, wobei sich die Aufbewahrung in dunklen Glasampullen empfiehlt.

Infusionslösungen sind stets frisch durch Zuspritzen der Ampullenkonfektion in die Träger-Infusionslösungen zuzubereiten.

Bei sämtlichen anderen Zubereitungsformen ist bei der Auswahl von Hilfs- und Füllstoffen darauf zu achten, daß diese mit der freien SH-Gruppe des reduzierten Glutathions keine unerwünschte Reaktion eingehen können, so daß der Wirkstoff unverändert in der jeweiligen Zubereitungsform aufbereitet, gelagert und verabfolgt werden kann.

### Versuchsergebnisse

### In-vitro-Versuche

### 1.1) In-vitro-Versuch an menschlichen Erythrozyten zur Verbesserung und Normalisierung des Redoxverhaltens:

Von 20 Patienten mit unterschiedlichen Erkrankungen wurden jeweils 10 ml menschlichen Venenblutes entnommen und dessen Gerinnungsfähigkeit durch Zusatz von Heparin aufgehoben. Als Parameter für die optimale Erythrozytenfunktion wurde vor und nach der Inkubation mit 0,5 mg reduziertem Glutathion bei 37°C während eines Zeitraumes von 30 Minuten der Gehalt an intraerythrozytärem reduziertem Glutathion bestimmt.
n = 20 (Angabe des Mittelwertes und der Standardabweichung)

| | |
|---|---|
| Leerwert: | 1,50 ± 0,47 mU |
| Wert nach Inkubation: | 1,99 ± 0,15 mU |

### 1.2) In-vitro-Versuch an Zellensystemen menschlicher Erythrozyten:

Unter denselben Bedingungen wie bei vorstehend beschriebenem Versuch wurden die Enzyme Glukose-6-Phosphat-Dehydrogenase und Glutathion-Reduktase aus Erythrozyten von 22 Patienten in vitro untersucht.
n = 22 (Angabe des Mittelwertes und der Standardabweichung)

| Leerwerte: | |
|---|---|
| Glukose-6-Phasphat-Dehydrogenase | 148 ± 22 mU/ 10⁹ Erys. |
| Glutathion-Reduktase | 9,5 ± 0,8 U/ 10¹¹ Erys. |

| Werte nach Inkubation: | |
|---|---|
| Glukose-6-Phosphat-Dehydrogenase | 179 ± 16 mU/ 10⁹ Erys. |
| Glutathion-Reduktase | 11,3 ± 1,2 U/ 10¹¹ Erys. |

### In-vivo-Versuche

### 2.1) Tierversuche am Kaninchen zur Verbesserung und/oder zur Normalisierung des Redoxstatus des Blutes:

Bei 5 verschiedenen Kaninchen wurden in 30 Versuchen im venösen Blut folgende Parameter nach der Methode und mit dem Gerät VINCENT gemessen:
Ohm'scher Widerstand: r
Redoxpotential E_{H} bzw. Elektronenpotential: rH₂
pH-wert: pH.

Die Werte wurden im Ohrvenenblut nach definierter Sauerstoffmangel-Belastung gemessen.
n = 30 (Angabe des Mittelwertes und der Standardabweichung)
r = 199 ± 12 Ohm/cm³ Werte nach Belastung
pH = 7,38 ± 0,21 ohne Gabe von
rH₂ = 24,9 ± 2,3 reduziertem Glutathion
Danach wurde den Tieren reduziertes Glutathion in einer Dosis von 10 mg/kg Körpergewicht intravenös verabreicht.
5 - 10 Minuten nach der Applikation von reduziertem Glutathion wurden folgende Werte ermittelt:
r = 202 ± 14 Ohm/cm³ Werte nach Gabe von
pH = 7,15 ± 0,16 reduziertem Glutathion
rH₂ = 21,8 ± 0,95 und Belastung
Randbedingungen: Temperatur 20°C, Luftdruck 767 mm Hg.

### 2.2) Selbstversuch am Menschen zur Verbesserung und/oder zur Normalisierung des Redoxstatus des Blutes:

Im menschlichen Venenblut wurden nach definierter Sauerstoff-Mangel-Belastung die gleichen Parameter bestimmt wie im vorstehend geschilderten Versuch. Insgesamt wurden 50 Versuche an verschiedenen Tagen durchgeführt.
n = 50 (Angabe des Mittelwertes und der Standardabweichung)
r = 197 ± 14,5 Ohm/cm Werte nach Belastung
pH = 7,41 ± 0,18 ohne Gabe von
rH₂ = 26,3 ± 2,1 reduziertem Glutathion
Danach wurde-reduziertes Glutathion in einer Dosis von 600 mg langsam intravenös verabreicht.
10 Minuten nach der Applikation von reduziertem Glutathion wurden folgende Werte ermittelt:
r = 204 ± 12,3 Ohm/cm³ Werte nach Gabe von
pH = 7,23 ± 0,11 reduziertem Glutathion
rH₂ = 23,1 ± 1,4 und Belastung
Randbedingungen: Temperatur 20°C, Luftdruck 758 - 770 mm Kg.

### 2.3) Versuch an 8 gesunden Graumäusen nach Transplantation eines malignen Melanoms vom Menschen.

In Fig. 1 ist das Wachstum des Tumors in Abhängigkeit von der Zeit dargestellt.

Nach Überimpfung von je 10⁶ Zellen eines malignen Melanoms vom Menschen an n = 8 gesunde Graumäuse starben vier nicht mit reduziertem Glutathion behandelte Tiere (Kontrollgruppe) im Zeitraum von 8 - 14 Tagen. Vier Tiere, die drei mal wöchentlich mit je 250 mg reduziertem Glutathion behandelt wurden (Testgruppe) zeigten ein deutlich langsameres Tumorwachstum und überlebten bis zur siebten Woche.

### 2.4) Versuch an 40 gesunden Graumäusen nach Transplantation eines malignen Melanoms vom Menschen.

40 gesunden Graumäusen wurden je 10⁶ maligne Melanomzellen überimpft. 10 unbehandelte Kontrolltiere starben in Zeitraum von 8 - 12 Tagen.
30 Tiere wurden vom Beginn der Versuchsreihe an drei mal wöchentlich mit je 300 mg reduziertem Glutathion behandelt. Von diesen Tieren starben drei innerhalb 15 Tagen, weitere fünf innerhalb 20 Tagen und weitere sieben innerhalb 30 Tagen.
15 Tiere, das sind 50% der mit reduziertem Glutathion behandelten Tiere, überlebten den 30 Tage dauernden Beobachtungszeitraum nach Impfung mit regressiven Tumorzellen.

## Patentansprüche

1. Glutathion-Thiol-Derivat der Formel 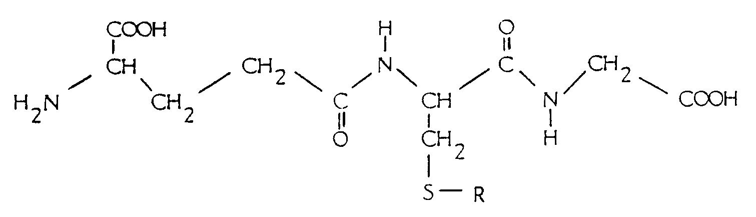 mit 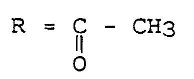 oder R = PO₃H₂
zur Verwendung als Arzneimittel.

2. Glutathion-Thiol-Derivat nach Anspruch 1
in Kombination mit zumindest einem Vitamin.

3. Glutathion-Thiol-Derivat nach Anspruch 1 oder 2
in Kombination mit zumindest einer Substanz, die ein Halbleiter-Element in anorganischer oder organischer Bindung enthält.

4. Glutathion-Thiol-Derivat nach Anspruch 1, 2 oder 3
in Kombination mit zumindest einer Aminosäure.

## Claims

1. A glutathione-thiol derivative of the formula 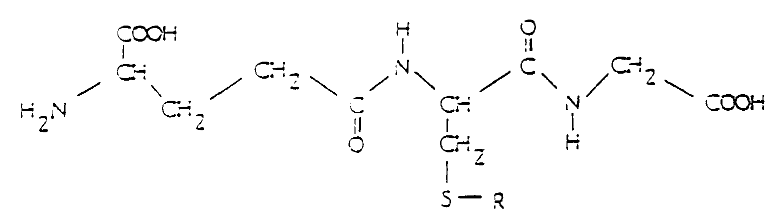 with 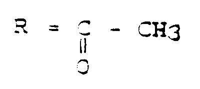 or R = PO₃H₂
for use as a medicament.

2. A glutathione-thiol derivative according to claim 1 in combination with at least one vitamin.

3. A glutathione-thiol derivative according to claim 1 or 2 in combination with at least one substance which contains an inorganically or organically bound semiconductor element.

4. A glutathione-thiol derivative according to claim 1, 2 or 3 in combination with at least one amino acid.

## Revendications

1. Dérivé glutathion-thiol de formule 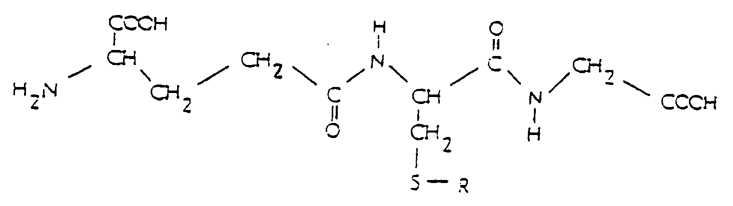 avec 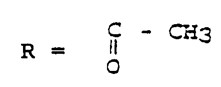 ou R = PO₃H₂
pour utilisation en tant que médicament.

2. Dérivé glutathion-thiol selon la revendication 1, en combinaison avec au moins une vitamine.

3. Dérivé glutathion-thiol selon la revendication 1 ou 2, en combinaison avec au moins une substance, qui contient un élément semi-conducteur dans un liant minéral ou organique.

4. Dérivé glutathion-thiol selon la revendication 1, 2 ou 3, en combinaison avec au moins un acide aminé.
